# EUROPEAN PATENT SPECIFICATION

(11) **EP 3 503 855 B1**
(45) Date of publication and mention of the grant of the patent: **29.12.2021**
(21) Application number: 17755013.4
(22) Date of filing: 11.08.2017
(51) Int. Cl.: A61F 13/00, A61F 13/02, A61K 9/70

(54) **WOUND DRESSING WITH ANALGESIC BORDER ADHESIVE**
WUNDVERBAND MIT ANALGETISCHEM RANDKLEBER
PANSEMENT AVEC ADHÉSIF DE BORDURE ANALGÉSIQUE

(30) Priority: 26.08.2016 US 201662380079 P
(43) Date of publication of application: 03.07.2019
(73) Proprietor: KCI Licensing, Inc., San Antonio, TX 78265-9508 (US); Systagenix Wound Management, Limited, Beehive Ring Road, Gatwick Airport, West Sussex RH6 0PA (GB)
(72) Inventor: LOCKE, Christopher, Brian, Bournemouth BH9 3SD (GB); PIGG, William, Elvington Yorkshire YO41 4DW (GB)
(74) Representative: Simmons & Simmons
(86) International application number: PCT/US2017/046632
(87) International publication number: WO 2018/038949

(56) References cited:
- EP-A1- 2 981 235
- WO-A1-2015/130608
- US-A1- 2006 172 002
- US-A1- 2010 318 052
- US-A1- 2012 323 191
- US-A1- 2014 221 907
- US-A1- 2016 045 376
- US-A1- 2016 120 706

## Description

### TECHNICAL FIELD

The invention set forth in the appended claims relates generally to tissue treatment, and more particularly, but without limitation, to wound dressings with an analgesic border adhesive.

### BACKGROUND

Many factors can contribute to pain associated with wound care. In addition to any pain caused by the wound itself, discomfort can also be caused by wound dressings in some cases. For example, some dressings may be adhered to skin adjacent to a wound, and intentional or inadvertent movement of a dressing can pull on the skin. Removal of a dressing can also be painful, sometimes even tearing skin.

Treatment of wounds with reduced pressure systems can also be complicated by pain associated with wound dressings. Treatment of wounds or other tissue with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times. However, contraction of a wound edge can also cause discomfort, particularly when pressure on the wound edge changes. US 2012/0323191 A1 and US 2006/0172002 A1 concern adhesive patches. WO 2015/130608 A1 concerns a medical dressing including a support material layer, a backing layer, and an adhesive. US 2016/0120706 A1 concerns sealing compositions for negative pressure treatment systems and wound dressing systems. WO 2014/161548 A1 concerns a medical dressing comprising an absorbent adhesive layer having a pharmaceutically active agent incorporated. US 2010/0318052 A1 concerns a conformable medical dressing with self supporting substrate.

While the clinical benefits of wound care and negative-pressure therapy are widely known, improvements to therapy systems, components, and processes may benefit healthcare providers and patients.

### BRIEF SUMMARY

The invention provides a wound dressing according to claim 1. Further embodiments are set out in the dependent claims. Illustrative embodiments are also provided to enable a person skilled in the art to make and use the claimed subject matter.

For example, in some embodiments, a wound dressing may comprise an adhesive border that contains a pain-reducing agent, such as ibuprofen. The wound interface layer may be a foam, that provides a drug-blocking layer between the analgesic adhesive and the wound interface layer. The drug-blocking layer can prevent the analgesic from being delivered to a wound, while allowing delivery to the periwound skin.

In other example embodiments, the dressing may be suitable for negative-pressure therapy and may comprise an analgesic adhesive. For example, a wound interface may be placed over a wound and covered by a first drape. A second drape with an analgesic adhesive may be placed over the first drape, and the analgesic adhesive applied to the periwound skin. The analgesic may be delivered to the periwound skin from the second drape, while the first drape prevents delivery of the analgesic to the wound. In some embodiments, the drug-delivery region may be colored or otherwise distinguished from other regions to facilitate proper placement of the dressing.

Other embodiments may comprise an analgesic tape or strips of film with an analgesic adhesive on one side. For example, an analgesic adhesive may be applied to strips of a polyurethane drape, which could be used to assemble an analgesic frame around a wound prior to applying a cover over the wound. The cover may have a conventional acrylic adhesive in some examples, which may be applied to the drape strips instead of the periwound skin.

The therapeutic composition may be a topical analgesic. The therapeutic composition may be dissolved into the adhesive, and preferably constitutes at least 20 percent of the dermal interface. In some embodiments, the therapeutic composition may comprise at least 30 percent of the dermal interface.

In some embodiments, the dermal interface may be disposed only in the border region, to the exclusion of the interior region of the backing layer. Additionally or alternatively, the barrier layer may be disposed over the dermal interface within the interior region, leaving only a margin of the dermal interface exposed.

The wound interface may be coupled to or adapted to be disposed adjacent to the interior region of the backing layer, or the barrier layer in some embodiments. Preferably the wound interface is positioned to leave the dermal interface (or a margin area of the dermal interface) exposed for application to periwound epidermis.

The dressing may be suitable for negative-pressure therapy in some embodiments. For example, an apparatus for providing negative-pressure therapy may comprise such a dressing with a tissue interface. The tissue interface may be fluidly coupled or adapted to be fluidly coupled to a negative-pressure source, and to distribute negative-pressure across a tissue site.

In yet other configurations, the dermal interface may comprise or consist essentially of a roll or strips suitable for attaching a dressing to epidermis around a tissue site. In some embodiments, such a configuration may be provided as part of a dressing kit, for example.

Objectives, advantages, and a preferred mode of making and using the claimed subject matter may be understood best by reference to the accompanying drawings in conjunction with the following detailed description of illustrative embodiments.

### BRIEF DESCRIPTION OF THE DRAWINGS

Figure 1 is a functional block diagram of an example embodiment of a therapy system that can provide negative-pressure therapy in accordance with this specification;
Figures 2A-2B are schematic diagrams of an example embodiment of a dressing; and
Figures 3A-3B are schematic diagrams of another example embodiment of a dressing.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting. Reference(s) to "embodiment(s)" throughout the description which are not under the scope of the appended claims merely represent possible exemplary executions and are not part of the present invention.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

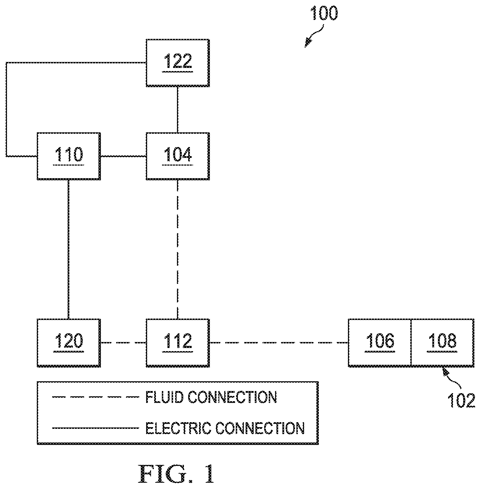

Figure 1 is a simplified functional block diagram of an example embodiment of a therapy system 100 that can provide negative-pressure therapy to a tissue site in accordance with this specification.

The term "tissue site" in this context broadly refers to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a negative-pressure supply, and may include or be configured to be coupled to a distribution component, such as a dressing. In general, a distribution component may refer to any complementary or ancillary component configured to be fluidly coupled to a negative-pressure supply in a fluid path between a negative-pressure supply and a tissue site. A distribution component is preferably detachable, and may be disposable, reusable, or recyclable. For example, a dressing 102 may be fluidly coupled to a negative-pressure source 104, as illustrated in Figure 1. A dressing may include a cover, a tissue interface, or both in some embodiments. The dressing 102, for example, may include a cover 106 and a tissue interface, such as a wound interface 108. A regulator or a controller, such as a controller 110, may also be coupled to the negative-pressure source 104.

In some embodiments, a dressing interface may facilitate coupling the negative-pressure source 104 to the dressing 102. For example, such a dressing interface may be a T.R.A.C.^{®} Pad or Sensa T.R.A.C.^{®} Pad available from KCI of San Antonio, Texas. The therapy system 100 may optionally include a fluid container, such as a container 112, coupled to the dressing 102 and to the negative-pressure source 104.

Additionally, the therapy system 100 may include sensors to measure operating parameters and provide feedback signals to the controller 110 indicative of the operating parameters. As illustrated in Figure 1, for example, the therapy system 100 may include a pressure sensor 120, an electric sensor 122, or both, coupled to the controller 110. The pressure sensor 120 may also be coupled or configured to be coupled to a distribution component and to the negative-pressure source 104.

Components may be fluidly coupled to each other to provide a path for transferring fluids (i.e., liquid and/or gas) between the components. For example, components may be fluidly coupled through a fluid conductor, such as a tube. A "tube," as used herein, broadly includes a tube, pipe, hose, conduit, or other structure with one or more lumina adapted to convey a fluid between two ends. Typically, a tube is an elongated, cylindrical structure with some flexibility, but the geometry and rigidity may vary. In some embodiments, components may also be coupled by virtue of physical proximity, being integral to a single structure, or being formed from the same piece of material. Moreover, some fluid conductors may be molded into or otherwise integrally combined with other components. Coupling may also include mechanical, thermal, electrical, or chemical coupling (such as a chemical bond) in some contexts. For example, a tube may mechanically and fluidly couple the dressing 102 to the container 112 in some embodiments.

In general, components of the therapy system 100 may be coupled directly or indirectly. For example, the negative-pressure source 104 may be directly coupled to the controller 110, and may be indirectly coupled to the dressing 102 through the container 112.

The fluid mechanics of using a negative-pressure source to reduce pressure in another component or location, such as within a sealed therapeutic environment, can be mathematically complex. However, the basic principles of fluid mechanics applicable to negative-pressure therapy are generally well-known to those skilled in the art, and the process of reducing pressure may be described illustratively herein as "delivering," "distributing," or "generating" negative pressure, for example.

In general, exudates and other fluids flow toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications (such as by substituting a positive-pressure source for a negative-pressure source) and this descriptive convention should not be construed as a limiting convention.

"Negative pressure" generally refers to a pressure less than a local ambient pressure, such as the ambient pressure in a local environment external to a sealed therapeutic environment provided by the dressing 102. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. Similarly, references to increases in negative pressure typically refer to a decrease in absolute pressure, while decreases in negative pressure typically refer to an increase in absolute pressure. While the amount and nature of negative pressure applied to a tissue site may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -75 mm Hg (-9.9 kPa) and -300 mm Hg (-39.9 kPa).

A negative-pressure supply, such as the negative-pressure source 104, may be a reservoir of air at a negative pressure, or may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components, such as sensors, processing units, alarm indicators, memory, databases, software, display devices, or user interfaces that further facilitate therapy. For example, in some embodiments, the negative-pressure source 104 may be combined with the controller 110 and other components into a therapy unit. A negative-pressure supply may also have one or more supply ports configured to facilitate coupling and de-coupling the negative-pressure supply to one or more distribution components.

The wound interface 108 can be generally adapted to contact a wound, and is preferably non-adherent or has a non-adherent surface adapted to contact a wound. The wound interface 108 may be partially or fully in contact with the tissue site. If the tissue site is a wound, for example, the wound interface 108 may fill the wound, or may be placed over the wound. The wound interface 108 may take many forms, and may have many sizes, shapes, or thicknesses depending on a variety of factors, such as the type of treatment being implemented or the nature and size of a tissue site. For example, the size and shape of the wound interface 108 may be adapted to the contours of deep and irregular shaped tissue sites. Moreover, any or all of the surfaces of the wound interface 108 may have projections or an uneven, course, or jagged profile that can induce strains and stresses on a tissue site, which can promote granulation at the tissue site.

In some embodiments, the wound interface 108 may be a manifold. A "manifold" in this context generally includes any substance or structure providing a plurality of pathways adapted to collect or distribute fluid across a tissue site under pressure. For example, a manifold may be adapted to receive negative pressure from a source and distribute negative pressure through multiple apertures across a tissue site, which may have the effect of collecting fluid from across a tissue site and drawing the fluid toward the source. In some embodiments, the fluid path may be reversed or a secondary fluid path may be provided to facilitate delivering fluid across a tissue site.

In some illustrative embodiments, the pathways of a manifold may be interconnected to improve distribution or collection of fluids across a tissue site. In some illustrative embodiments, a manifold may be a porous foam material having interconnected cells or pores. For example, cellular foam, open-cell foam, reticulated foam, porous tissue collections, and other porous material such as gauze or felted mat generally include pores, edges, and/or walls adapted to form interconnected fluid channels. Liquids, gels, and other foams may also include or be cured to include apertures and fluid pathways. In some embodiments, a manifold may additionally or alternatively comprise projections that form interconnected fluid pathways. For example, a manifold may be molded to provide surface projections that define interconnected fluid pathways.

The average pore size of a foam may vary according to needs of a prescribed therapy. For example, in some embodiments, the wound interface 108 may be a foam having pore sizes in a range of 400-600 microns. The tensile strength of the wound interface 108 may also vary according to needs of a prescribed therapy. For example, the tensile strength of a foam may be increased for instillation of topical treatment solutions. In one non-limiting example, the wound interface 108 may be an open-cell, reticulated polyurethane foam such as GranuFoam^{®} dressing or VeraFlo^{®} foam, both available from Kinetic Concepts, Inc. of San Antonio, Texas.

The wound interface 108 may be either hydrophobic or hydrophilic. In an example in which the wound interface 108 may be hydrophilic, the wound interface 108 may also wick fluid away from a tissue site, while continuing to distribute negative pressure to the tissue site. The wicking properties of the wound interface 108 may draw fluid away from a tissue site by capillary flow or other wicking mechanisms. An example of a hydrophilic foam is a polyvinyl alcohol, open-cell foam such as V.A.C. WhiteFoam^{®} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

The wound interface 108 may further promote granulation at a tissue site when pressure within the sealed therapeutic environment is reduced. For example, any or all of the surfaces of the wound interface 108 may have an uneven, coarse, or jagged profile that can induce microstrains and stresses at a tissue site if negative pressure is applied through the wound interface 108.

In some embodiments, the wound interface 108 may be constructed from bioresorbable materials. Suitable bioresorbable materials may include, without limitation, a polymeric blend of polylactic acid (PLA) and polyglycolic acid (PGA). The polymeric blend may also include without limitation polycarbonates, polyfumarates, and capralactones. The wound interface 108 may further serve as a scaffold for new cell-growth, or a scaffold material may be used in conjunction with the wound interface 108 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

A controller, such as the controller 110, may be a microprocessor or computer programmed to operate one or more components of the therapy system 100, such as the negative-pressure source 104. In some embodiments, for example, the controller 110 may be a microcontroller, which generally comprises an integrated circuit containing a processor core and a memory programmed to directly or indirectly control one or more operating parameters of the therapy system 100. Operating parameters may include the power applied to the negative-pressure source 104, the pressure generated by the negative-pressure source 104, or the pressure distributed to the wound interface 108, for example. The controller 110 is also preferably configured to receive one or more input signals, such as a feedback signal, and programmed to modify one or more operating parameters based on the input signals.

Sensors, such as the pressure sensor 120 or the electric sensor 122, are generally known in the art as any apparatus operable to detect or measure a physical phenomenon or property, and generally provide a signal indicative of the phenomenon or property that is detected or measured. For example, the pressure sensor 120 and the electric sensor 122 may be configured to measure one or more operating parameters of the therapy system 100. In some embodiments, the pressure sensor 120 may be a transducer configured to measure pressure in a pneumatic pathway and convert the measurement to a signal indicative of the pressure measured. In some embodiments, for example, the pressure sensor 120 may be a piezoresistive strain gauge. The electric sensor 122 may optionally measure operating parameters of the negative-pressure source 104, such as the voltage or current, in some embodiments. Preferably, the signals from the pressure sensor 120 and the electric sensor 122 are suitable as an input signal to the controller 110, but some signal conditioning may be appropriate in some embodiments. For example, the signal may need to be filtered or amplified before it can be processed by the controller 110. Typically, the signal is an electrical signal, but may be represented in other forms, such as an optical signal.

The container 112 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

In operation, the wound interface 108 may be placed within, over, on, or otherwise proximate to a tissue site. The cover 106 may be placed over the wound interface 108 and sealed to an attachment surface near the tissue site. For example, the cover 106 may be sealed to undamaged epidermis peripheral to a tissue site. Thus, the dressing 102 can provide a sealed therapeutic environment proximate to a tissue site, substantially isolated from the external environment, and the negative-pressure source 104 can reduce the pressure in the sealed therapeutic environment. Negative pressure applied across the tissue site through the wound interface 108 in the sealed therapeutic environment can induce macrostrain and microstrain in the tissue site, as well as remove exudates and other fluids from the tissue site, which can be collected in the container 112.

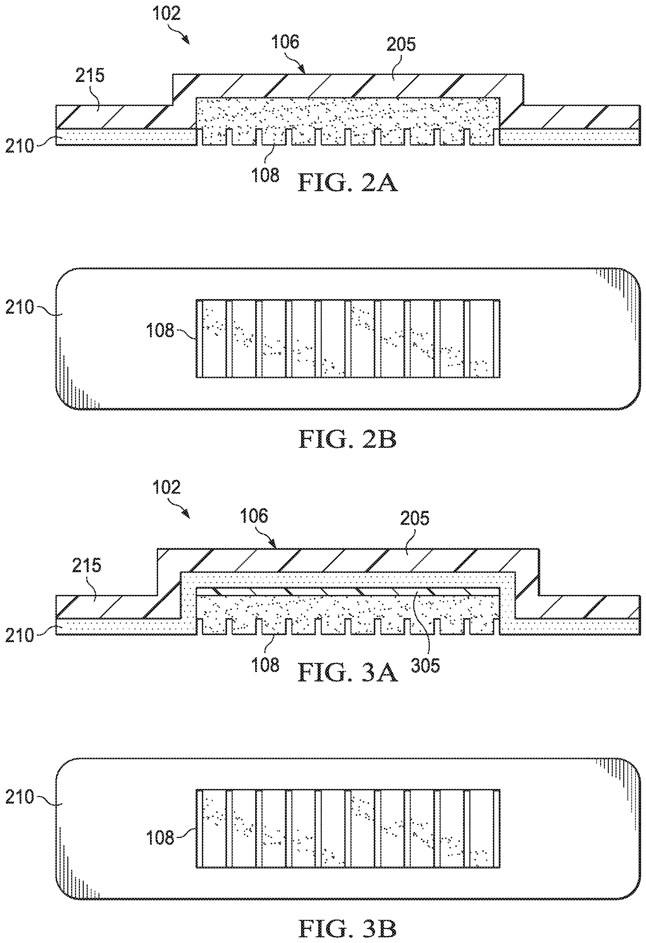

Figures 2A-2B are schematic diagrams of an example embodiment of the dressing 102. As illustrated in the example of Figure 2A, some embodiments of the cover 106 may comprise a backing layer 205 and a dermal interface 210.

In some embodiments, the backing layer 205 may provide a bacterial barrier and protection from physical trauma. The backing layer 205 may also be constructed from a material that can reduce evaporative losses and provide a fluid seal between two components or two environments, such as between a therapeutic environment and a local external environment. The backing layer 205 may be, for example, an elastomeric film or membrane that can provide a seal adequate to maintain a negative pressure at a tissue site for a given negative-pressure source. The backing layer 205 may have a high moisture-vapor transmission rate (MVTR) in some applications. For example, the MVTR may be at least 300 g/m^2 per twenty-four hours in some embodiments. In some example embodiments, the backing layer may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained.

The dermal interface 210 may be used to attach the cover 106 to a periwound surface, such as undamaged epidermis surrounding a wound. The dermal interface 210 may comprise or consist essentially of an adhesive and a therapeutic composition in some embodiments. For example, the dermal interface 210 may comprise a polymer adhesive in which the therapeutic composition is dissolved. The therapeutic composition may comprise at least 20 percent by weight of the dermal interface 210 in some embodiments, and preferably at least 30 percent by weight in some embodiments. Various types of therapeutic compositions may be suitable, including topical analgesic compositions. A non-steroidal anti-inflammatory drug (NSAID) may be particularly advantageous for some embodiments.

In some embodiments, the dermal interface 210 may include a polymer adhesive formed form a therapeutic composition, a solubilizing prepolymer, and a cross-linking group. As previously mentioned, the therapeutic composition may include one or more anti-inflammatory or pain-relieving drugs, for example an NSAID. For example, in some embodiments, the drug may be ibuprofen (isobutylphenylpropanoic acid) or aspirin (methyl salicylate), or a salt of one or more of the drugs. The therapeutic composition may be dissolved or associated with the solubilizing prepolymer. Example solubilizing prepolymers may include glycols such as polyethylene glycol or polyethylene oxide, alcohols such as polyvinyl alcohol and associated copolymers, acrylics such as acrylic and methacrylic acids and associated salts and esters, esters such as vinyl esters, for example vinyl acetate, and aromatic esters, for example phthalates such as polyethylene terephthalate, amides and amines such as pyrrolidone and associated copolymers, and polyurethanes.

The solubilizing prepolymer, which may include the associated therapeutic composition, may react with the cross-linking group to form a polymer with cross-linking groups at the ends of the polymer chains. In some embodiments, the cross-linking groups may react with moisture to cross-link. Additionally, the cross-linking end groups may further react to extend the length of the polymer chains as well as further cross-link the polymers. In some embodiments, the cross-linking group may comprise or consist essentially of silyl groups, such as methoxy silane, and/or isocyanates. Some example embodiments of cross-linking groups may comprise a polymer having silicon-containing chemical groups which may be capable of hydrolyzing in the presence of moisture to form siloxane linkages through a condensation reaction. Such moisture cross-linking polymers may include silyl-modified polymers (SMP), in which a polymer backbone of a polymer such as a polyether, polyurethane, polyester, or other type of polymer is modified on one or both ends of the backbone and possibly also with side branches with moisture-reactive silyl groups. One subclass of silyl-modified polymers are MS-Polymers^{®}, commercially available from Kaneka company of Japan. Additional details regarding silyl-modified polymers may be found in US Patent Publication No. 2015/0210908 A1 to Vosters et al. In some preferred embodiments, the solubilizing prepolymer may comprise polyethylene oxide (PEO) and the cross-linking group may comprise silyl groups in order to form a polymer adhesive that is strong and that has silicone gel-like properties when in contact with skin tissue. An additional benefit of selecting a relatively low-viscosity prepolymer, such as PEO, may be that little or no plasticizer is required, thus resulting in a low-residue adhesive for use on the skin.

In some embodiments, the dermal interface 210 may be disposed in a border or margin of the backing layer 205, to the exclusion of an interior region of the backing layer 205. For example, the dermal interface 210 may be disposed in a border region 215 of the backing layer 205, as illustrated in the example of Figure 2A. The border region 215 generally encompasses an area of the backing layer 205 proximate to the outer edge of the backing layer 205, and preferably circumscribes the interior region.

Figures 2A-2B also illustrate an example embodiment of the wound interface 108, which may be disposed interior to the dermal interface 210. The wound interface 108 preferably has a size and shape similar to the interior region of the backing layer 205.

In use, the wound interface 108 is preferably positioned between a wound and the interior region of the backing layer 205, and the dermal interface 210 may be positioned on the periwound so that no portion of the dermal interface 210 is in contact with the wound.

Figures 3A-3B are schematic diagrams of another example embodiment of the dressing 102. In the example of Figure 3A, the dermal interface 210 may coat an entire surface of the backing layer 205, including the border region 215. A barrier layer 305 may be disposed interior to the border region 215 so that the portion of the dermal interface 210 interior to the border region 215 is disposed between the backing layer 205 and the barrier layer 305. The barrier layer 305 generally comprises or consists of a material or composition that is adapted to block or contain any therapeutic composition in the dermal interface 210. For example, in some embodiments, the barrier layer 305 may comprise or consist essentially of a polyurethane film. As illustrated in the example of Figure 3, in some embodiments the barrier layer 305 may be disposed between the dermal interface 210 and other layers of the dressing 102, such as the wound interface 108.

In use, the barrier layer 305 is preferably disposed between a wound and the dermal interface 210 to prevent delivery of any therapeutic composition in the dermal interface 210 to the wound. The dermal interface 210, the barrier layer 305, or both may be marked or color-coded to facilitate proper placement over the wound.

The dermal interface 210 may also be provided in other configurations. For example, the dermal interface 210 may be provided as a roll or strips suitable for attaching a dressing to epidermis around a tissue site. For example, in some embodiments, a periwound tape may comprise a layer of the dermal interface 210 coupled to a backing layer. The backing layer may comprise or consist essentially of material having properties similar to the backing layer 205, such as a polyurethane drape.

The periwound tape may be provided as part of a dressing kit, for example, as a roll or in strips. In some embodiments, the kit may additionally include a wound interface, such as the wound interface 108, and a cover or drape, such as the cover 106. In use, the periwound tape may be cut, sized, or otherwise manipulated to assemble an frame or window around a tissue site prior to applying the cover. The cover may have a conventional acrylic adhesive in some examples, which may be applied to the backing layer of the periwound tape instead of the periwound epidermis.

The systems, apparatuses, and methods described herein may provide significant advantages. For example, the dermal interface 210 can deliver an analgesic to epidermis around a wound to reduce pain during wear and removal of a dressing, without delivering the analgesic to the wound. The dermal interface 210 may be particularly beneficial when used with negative-pressure therapy, since pressure changes can contract the cover 106 and cause linear strain on periwound tissue.

While shown in a few illustrative embodiments, a person having ordinary skill in the art will recognize that the systems, apparatuses, and methods described herein are susceptible to various changes and modifications. Moreover, descriptions of various alternatives using terms such as "or" do not require mutual exclusivity unless clearly required by the context, and the indefinite articles "a" or "an" do not limit the subject to a single instance unless clearly required by the context.

Components may be also be combined or eliminated in various configurations for purposes of sale, manufacture, assembly, or use. For example, in some configurations the cover 106, the wound interface 108, or both may be eliminated or separated from other components for manufacture or sale. In some embodiments, the dermal interface 210 may be applied over other adhesives. For example, the dermal interface 210 may be applied over an acrylic adhesive on the cover 106. Additionally or alternatively, some or all of the therapeutic composition of the dermal interface 210 may be encapsulated and released by applying pressure to the dermal interface 210. Moreover, use of the dermal interface 210 is not limited to dressings for negative-pressure therapy.

The appended claims set forth novel and inventive aspects of the subject matter described above, but the claims may also encompass additional subject matter not specifically recited in detail.

## Claims

1. A wound dressing (102), comprising:
a backing layer (205) comprising an interior region and a border region (215) around the interior region;
a dermal interface (210) disposed in the border region, the dermal interface comprising an adhesive and a therapeutic composition;
a wound interface (108) disposed in the interior region; and
**characterized by** a barrier layer (305) disposed in the interior region between the dermal interface (210) and the wound interface (108).

2. The wound dressing of claim 1, wherein the therapeutic composition comprises at least 20% by weight of the dermal interface.

3. The wound dressing of claim 1, wherein the therapeutic composition comprises at least 30% by weight of the dermal interface.

4. The wound dressing of claim 1, wherein the barrier layer comprises a polyurethane film.

5. The wound dressing of any of claims 1-4, wherein the therapeutic composition comprises a topical analgesic.

6. The wound dressing of claim 5, wherein the therapeutic composition is a non-steroidal anti-inflammatory drug.

7. The wound dressing of claim 1, wherein:
the backing layer is a first polyurethane film; and
the therapeutic composition comprises at least 30% by weight of the dermal interface.

## Patentansprüche

1. Wundverband (102), der umfasst:
eine Rückseitenschicht (205), die einen Innenbereich und einen Randbereich (215) um den Innenbereich umfasst;
eine Hautschnittfläche (210), die in dem Randbereich angeordnet ist, wobei die Hautschnittfläche einen Klebstoff und eine therapeutische Zusammensetzung umfasst;
eine Wundschnittfläche (108), die in dem Innenbereich angeordnet ist; und
**gekennzeichnet durch** eine Sperrschicht (305), die in dem Innenbereich zwischen der Hautschnittfläche (210) und der Wundschnittfläche (108) angeordnet ist.

2. Wundverband nach Anspruch 1, wobei die therapeutische Zusammensetzung mindestens 20 Gew.-% der Hautschnittfläche umfasst.

3. Wundverband nach Anspruch 1, wobei die therapeutische Zusammensetzung mindestens 30 Gew.-% der Hautschnittfläche umfasst.

4. Wundverband nach Anspruch 1, wobei die Sperrschicht eine Polyurethanfolie umfasst.

5. Wundverband nach einem der Ansprüche 1 bis 4, wobei die therapeutische Zusammensetzung ein topisches Analgetikum umfasst.

6. Wundverband nach Anspruch 5, wobei die therapeutische Zusammensetzung ein nicht-steroidales entzündungshemmendes Arzneimittel ist.

7. Wundverband nach Anspruch 1, wobei:
die Rückseitenschicht eine erste Polyurethanfolie ist; und
die therapeutische Zusammensetzung mindestens 30 Gew.-% der Hautschnittfläche umfasst.

## Revendications

1. Pansement (102) comprenant :
une couche de support (205) comprenant une région intérieure et une région de bordure (215) autour de la région intérieure ;
une interface dermique (210) disposée dans la région de bordure, l'interface dermique comprenant un adhésif et une composition thérapeutique ;
une interface de plaie (108) disposée dans la région intérieure ; et
**caractérisé par** une couche barrière (305) disposée dans la région intérieure entre l'interface dermique (210) et l'interface de plaie (108).

2. Pansement selon la revendication 1, dans lequel la composition thérapeutique comprend au moins 20 % en poids de l'interface dermique.

3. Pansement selon la revendication 1, dans lequel la composition thérapeutique comprend au moins 30 % en poids de l'interface dermique.

4. Pansement selon la revendication 1, dans lequel la couche barrière comprend un film de polyuréthane.

5. Pansement selon l'une quelconque des revendications 1-4, dans lequel la composition thérapeutique comprend un analgésique topique.

6. Pansement selon la revendication 5, dans lequel la composition thérapeutique est un médicament anti-inflammatoire non stéroïdien.

7. Pansement selon la revendication 1, dans lequel :
la couche de support est un premier film de polyuréthane ; et
la composition thérapeutique comprend au moins 30% en poids de l'interface dermique.
